# FASCICULE DE BREVET EUROPEEN

(11) **EP 3 560 916 B1**
(45) Date de publication et mention de la délivrance du brevet: **23.02.2022**
(21) Numéro de dépôt: 19170415.4
(22) Date de dépôt: 19.04.2019
(51) Int. Cl.: A61K 8/49, A61Q 5/02, A61Q 11/00, A61Q 19/10, C07D 307/68, C11D 1/04, C11D 1/66, C07D 307/56

(54) **PROCÉDÉ DE SYNTHÈSE DE 5-DIALKYLACÉTAL-2-FUROATES D'ALKYLE ET LEUR UTILISATION DANS LA PRÉPARATION D'AGENTS TENSIOACTIFS BIOSOURCÉS**
SYNTHESEVERFAHREN VON 5-DIALKYLAZETAL-2-FUROATEN VON ALKYL, UND IHRE VERWENDUNG FÜR DIE ZUBEREITUNG VON BIOBASIERTEN TENSIDEN
METHOD FOR SYNTHESISING ALKYL 5-DIALKYLACETAL-2-FUROATE AND USE THEREOF IN THE PREPARATION OF BIOSOURCED SURFACTANT AGENTS

(30) Priorité: 24.04.2018 FR 1853588
(43) Date de publication de la demande: 30.10.2019
(73) Titulaire: Surfactgreen, 60201 Compiegne Cedex (FR); Ecole Nationale Superieure de Chimie de Rennes, 35700 Rennes Cedex (FR); CENTRE NATIONAL DE LA RECHERCHE SCIENTIFIQUE, 75016 Paris (FR)
(72) Inventeur: BENVEGNU, Thierry, 35000 RENNES (FR); RENAULT, Louise, 61200 ARGENTAN (FR); DIVET, Pierre-Yves, 92200 NEUILLY-SUR-SEINE (FR); ROUSSEL, Xavier, 72000 LE MANS (FR)
(74) Mandataire: Renard, Emmanuelle

(56) Documents cités:
- WO-A1-2004/037808
- WO-A1-2013/049711
- WO-A1-2014/182171
- WO-A1-2017/030668
- WO-A1-2017/098175
- FRITS VAN DER KLIS , DR. JACCO VAN HAVEREN , DR. DAAN S. VAN ES , PROF. DR. JOHANNES H. BITTER: "Synthesis of Furandicarboxylic Acid Esters From Nonfood Feedstocks Without Concomitant Levulinic Acid Formation", CHEMISTRY AND SUSTAINABILITY, vol. 10, 26 janvier 2017 (2017-01-26), pages 1460-1468, XP002786857, DOI: 10.1002/cssc.201700051
- CASANOVA O ET AL: "Biomass into chemicals: One pot-base free oxidative esterification of 5-hydroxymethyl-2-furfural into 2,5-dimethylfuroate with gold on nanoparticulated ceria", JOURNAL OF CATALYSIS, ACADEMIC PRESS, DULUTH, MN, US, vol. 265, no. 1, 2009, pages 109-116, XP026157587, ISSN: 0021-9517, DOI: 10.1016/J.JCAT.2009.04.019 [extrait le 2009-05-28]

## Description

### OBJET DE L'INVENTION

La présente invention concerne un procédé de synthèse de 5-dialkylacétal-2-furoates d'alkyle, ainsi qu'une composition susceptible d'être obtenue suivant ce procédé. Elle concerne également des sels d'acide 5-dialkylacétal-2-furoïque susceptibles d'être préparés à partir de ces composés et qui sont utilisables comme agents tensioactifs.

### ARRIERE-PLAN DE L'INVENTION

Les synthons biosourcés furaniques tels que le furfural et l'hydroxyméthylfurfural (HMF) sont qualifiés de molécules "plateformes" du fait de la multitude de dérivés et d'applications auxquels ils conduisent par transformations chimiques. Ils sont produits industriellement par déshydratation des sucres en C5 comme le xylose (furfural) ou en C6 comme le fructose (5-HMF). Ces sucres sont présents dans des matières premières issues de la biomasse comme par exemple dans les ressources lignocellulosiques. En particulier, le 5-HMF est une molécule plateforme, particulièrement intéressante pour l'industrie chimique. D'ailleurs en 2010, elle avait été identifiée comme l'une des 10 molécules chimiques clés par le DOE, ministère américain de l'Énergie. Parmi ses dérivés possibles, on peut citer le 2,5-diméthylfurane (DMF) qui peut être utilisé comme biocarburant à la place de l'éthanol ou l'acide 2,5-furane dicarboxylique (FDCA) qui est proposé comme remplaçant à l'acide téréphtalique dans le PET. Certains de ces composés comme par exemple le HMF possèdent des propriétés physicochimiques peu adaptées à des développements industriels comme par exemple une miscibilité élevée en milieu aqueux et une instabilité chimique. L'accès à de nouvelles molécules plateformes plus stables et non miscibles en phase aqueuse présente donc un intérêt important.

D'autre part, l'obtention de formes dissymétriques comportant deux groupes fonctionnels différents en positions 2 et 5 du cycle furanique, respectivement de type acide carboxylique (ou ses dérivés esters) et aldéhyde (ou ses dérivés acétals) reste difficile à réaliser à partir de polysaccharides ou par transformation chimique du HMF. En effet, les réactions d'oxydation sélective de l'alcool primaire du HMF en acide carboxylique en présence de la fonction aldéhyde ne sont pas aisées. De ce fait, la chimie de ces dérivés 2-furoïques comportant une fonction aldéhyde ou acétal en position 5 a été à ce jour peu développée. Dans ce contexte, la mise au point d'un procédé de production simple de molécules de type 5-dialkylacétal-2-furoates d'alkyle (DFA) pourrait ouvrir la voie à de nouvelles molécules plateformes. En effet, ces dérivés sont caractérisés par la présence des deux groupes fonctionnels carboxyle et aldéhyde protégés sous forme d'ester et de dialkylacétal. Cette double protection permet des transformations sélectives et de façon indépendante de chacune des fonctions, facilitant l'obtention de nouveaux dérivés furaniques en deux ou trois étapes seulement.

Dans la littérature, les stratégies décrites pour conduire à ces 5-dialkylacétal-2-furoates d'alkyle reposent généralement sur l'utilisation du 5-hydroxymethylfurfural (HMF) comme matière première et elles nécessitent une étape d'acétalisation de l'aldéhyde et une étape d'oxydation de l'alcool primaire, pouvant conduire à la formation de plusieurs produits secondaires résultant par exemple de réactions de dimérisation. Casanova et al. décrit par exemple un procédé de préparation du diméthylfuroate (DMF) à partir du HMF, en présence d'un catalyseur à base d'or et d'oxyde de cérium (Journal of Catalysis, 2009, vol. 265, 109-116). Une autre voie d'accès à ce type de structure a été décrite récemment (WO 2017/030668) en utilisant comme matières premières des dérivés de l'acide gluconique (acide déhydro-gluconique, DHG ; acide 2 keto-gluconique, 2KGA ; acide 5 keto-gluconique, 5KGA) mais l'accès à ces composés suppose la préparation préalable des précurseurs DHG, 2KGA et 5KGA via un procédé en trois étapes reposant sur (1) l'oxydation enzymatique ou la déshydrogénation enzymatique du glucose en 1,5-gluconolactone ; (2) l'hydrolyse de la 1,5-gluconolactone en gluconate ; et (3) la conversion enzymatique du gluconate en DHG, 2KGA ou 5KGA. La formation d'un précurseur de type 5KGA par isomérisation d'un acide uronique a été également décrite (WO 2014/182171 et ChemSusChem 2017, 10, 1460-1468), et nécessite des conditions particulières, notamment l'utilisation d'un sel de calcium.

Par conséquent, il existe un besoin de procédés plus efficaces, plus directs et plus simples à transposer à l'échelle industrielle pour produire des 5-dialkylacétal-2-furoates d'alkyle comme nouvelles molécules plateformes.

Un domaine permettant de valoriser ces synthons biosourcés est celui des tensioactifs à motif furanoïque. Les tensioactifs (ou agents de surface) sont largement utilisés dans divers domaines comme la cosmétique, la détergence, l'agrochimie, le BTP ou encore les peintures car ils possèdent des propriétés solubilisantes, mouillantes, détergentes ou émulsionnantes. Aujourd'hui, la plupart des tensioactifs sont issus de la pétrochimie. Cependant, face à l'épuisement annoncé des réserves de pétrole et l'augmentation avérée des gaz à effets de serre, l'utilisation de ressources renouvelables non fossiles apparait comme une véritable nécessité. Dans ce contexte de chimie verte, la recherche de tensioactifs biosourcés moins écotoxiques et biodégradables révèle un fort potentiel de croissance.

### RESUME DE L'INVENTION

De façon inopinée, les inventeurs ont mis en évidence qu'une modification des conditions d'un procédé décrit dans la littérature (WO 2017/098175) pour obtenir des tensioactifs osidiques, à savoir des alkylguloside uronates d'alkyle, à partir d'alginates et d'autres polysaccharides à motifs acide uronique permettait d'obtenir des 5-dialkylacétal-2-furoates d'alkyle utiles comme molécules plateformes et notamment pour l'obtention de tensioactifs biosourcés. Les inventeurs ont en outre démontré que les sels obtenus par saponification des 5-dialkylacétal-2-furoates d'alkyle, c'est-à-dire les sels d'acide 5-dialkylacétal-2-furoïque (WO 2004/037808), présentaient des propriétés tensioactives plus performantes que les sels décrits dans WO 2017/098175. En particulier, il s'avère que les performances des tensioactifs préparés sont comparables à celles d'un tensioactif anionique fort largement utilisé dans l'industrie, mais non biosourcé, le laurylsulfate de sodium (SLS).

Il est certes connu que ces polysaccharides peuvent conduire à des formes furaniques dans des conditions de dépolymérisation acides ou enzymatiques conduites à au plus 100°C (WO 2013/049711). Cependant, l'obtention de 5-dialkylacétal-2-furoates d'alkyle directement à partir de ces polysaccharides, sans isoler de produit intermédiaire ni avoir recours à une réaction d'oxydation, n'a pas été décrite à ce jour. Les inventeurs ont démontré que seul le recours à une température de réaction d'au moins 150°C permettait d'obtenir ces composés avec un rendement acceptable.

L'invention a ainsi pour objet un procédé de synthèse d'un 5-dialkylacétal-2-furoate d'alkyle de formule (I) : 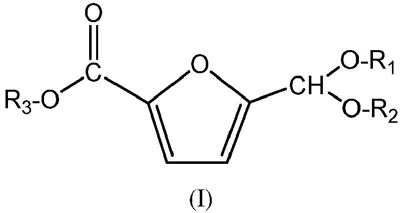 dans laquelle : R₁, R₂ et R₃ désignent chacun indépendamment un groupe alkyle ou alcényle en C₁-C₂₂,
caractérisé en ce qu'il comprend une étape de transformation d'un ou plusieurs acides uroniques par réaction avec au moins un alcool de formule R-OH où R désigne un groupe alkyle ou alcényle en C₁-C₂₂, en présence d'au moins un catalyseur acide, le milieu réactionnel étant porté à une température de 150 à 180°C.

La présente invention a encore pour objet des sels d'acide 5-dialkylacétal-2-furoïque de formule (II) : 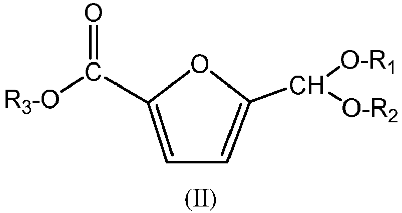 dans laquelle : R₁ et R₂ désignent chacun indépendamment un groupe alkyle ou alcényle en C₁-C₂₂ ; et R₃ désigne un atome de métal alcalin ou de métal alcalino-terreux ou un groupe ammonium quaternaire de formule (III) :

-N⁺-R₄R₅R₆R₇ (III)

où R₄, R₅, R₆ et R₇ désignent chacun indépendamment un atome d'hydrogène, un groupe alkyle en C₁-C₆ ou un groupe hydroxyalkyle en C₁-C₆.

Elle a également pour objet l'utilisation de ces sels comme agents tensioactifs, ainsi qu'une composition détergente renfermant ces sels.

Les 5-dialkylacétal-2-furanoates d'alkyle obtenus selon l'invention ne sont pas miscibles avec l'eau et présentent une bonne stabilité chimique. Ils constituent des molécules plateformes qui peuvent être utilisées dans de nombreux domaines d'application, et en particulier pour la synthèse de tensioactifs. Ces molécules présentent en effet une fonction ester et une fonction acétal qui peuvent être sélectivement déprotégées, respectivement par saponification ou par hydrolyse acide, pour aboutir à des composés présentant une fonction acide carboxylique ou aldéhyde, respectivement.

Ils sont obtenus suivant un procédé qui peut être mis en œuvre en récipient unique (ou "one-pot" en anglais), en l'absence de solvant organique, à partir de réactifs biodégradables.

### DESCRIPTION DETAILLEE

### Définitions

Dans le contexte de cette description, par "alkyle", on entend un groupe hydrocarboné aliphatique saturé, linéaire ou ramifié, renfermant de 1 à 22 atomes de carbone, notamment un groupe méthyle, éthyle, propyle, butyle, isobutyle, hexyle, octyle, décyle, dodécyle, tétradécyle, hexadécyle ou octadécyle.

Par "alcényle", on entend un groupe hydrocarboné aliphatique insaturé, linéaire ou ramifié, renfermant de 2 à 22 atomes de carbone, notamment un groupe propényle, butényle, isobutényle, hexényle, octényle, décényle, dodécényle, tétradécényle, hexadécényle ou octadécényle.

Par "hydroxyalkyle", on entend un groupe alkyle substitué par au moins un groupe hydroxyle. Par "métal alcalin", on entend un élément chimique du premier groupe du tableau périodique, par exemple le sodium ou le potassium.

Par "métal alcalino-terreux", on entend un élément chimique du second groupe du tableau périodique, en particulier le magnésium ou le calcium.

Par « solvant ionique », on entend un liquide ionique, c'est-à-dire un sel ayant un point de fusion inférieur à 100°C et de préférence inférieur à 30°C, par exemple le chlorure de 1-butyl-3-méthylimidazolium [BMIM]Cl, le bromure de 1-butyl-3-méthylimidazolium [BMIM]Br, le méthylsulfate de tris-(2-hydroxyéthyl)méthylammonium (HEMA) et l'acétate de 1-éthyl-3-méthylimidazolium [EMIM]AcO ; ledit solvant ionique comprenant typiquement jusqu'à 10% d'eau.

Par « solvant eutectique », on entend des systèmes formés d'un mélange eutectique de bases ou d'acides de Lewis ou Brönsted qui peuvent contenir une variété d'espèces anioniques et/ou d'espèces cationiques. Les solvants eutectiques de première génération ont été basés sur des mélanges de sels d'ammonium quaternaire avec des donneurs de liaison d'hydrogène tels que les amines et les acides carboxyliques, notamment un sel d'ammonium quaternaire et (hydrate de) chlorure de métal.

La présente invention concerne un procédé permettant de préparer des 5-dialkylacétal-2-furoates d'alkyle à partir d'un ou plusieurs acides uroniques.

Dans une forme d'exécution de l'invention, ces acides uroniques sont constitués d'un hydrolysat d'oligo- ou polysaccharides comprenant un ou plusieurs motifs identiques ou différents d'acide uronique éventuellement salifiés. Avantageusement, les oligo- ou polysaccharides renferment au moins 40% molaire, de préférence au moins 70% molaire et, plus préférentiellement au moins 90% molaire de motifs acide uronique éventuellement associés à des motifs rhamnose, galactose et/ou glucose. Des exemples de tels oligo- ou polysaccharides sont les alginates, les oligoalginates, les polymannuronates, les oligomannuronates, les polyguluronates et les oligoguluronates (origine marine) ; les pectines ou oligopectines (origine terrestre) ; et les ulvanes ou oligoulvanes (origine marine).

Par « alginates », au sens de la présente invention, on entend des alginates raffinés et/ou semi-raffinés, par exemple obtenus selon le procédé de la demande internationale WO 2017/098175, à partir des espèces telles que *Ascophyllum, Durvillaea, Ecklonia, Laminaria, Lessonia, Macrocystis, Sargassum* et *Turbinaria.*

Par « oligoalginates » au sens de la présente invention, on entend les produits issus d'un traitement par voie enzymatique et/ou acide d'alginate, par exemple obtenus selon le procédé de la demande internationale WO 98/40511.

Par « polymannuronates » et « oligomannuronates » au sens de la présente invention, on entend des blocs homopolymériques d'acide β-D-mannuronique en partie sous forme de sels de sodium issus de la dépolymérisation d'alginates, par exemple selon le procédé de la demande internationale WO 03/099870.

Par « polyguluronates » et « oligoguluronates » au sens de la présente invention, on entend des blocs homopolymériques d'acide α-L-guluronique en partie sous forme de sels de sodium issus de la dépolymérisation d'alginates, par exemple selon le procédé de la demande internationale WO 03/099870.

Par « pectines » au sens de la présente invention, on entend par exemple les pectines naturelles, telles que les pectines hautement méthylées, les pectines faiblement méthylées, les pectines amidées.

Par « oligopectines » au sens de la présente invention, on entend des produits de dépolymérisation de la pectine en général obtenus par l'interaction de la pectinase et de la pectine chez les plantes ; les oligopectines étant des produits de dépolymérisation partielle des pectines (Demande JP 2012187042 ; Demande EP 2308989 ; Demande KR 20130070396 ; Demande UA 54330 ; Demande GB 792518 ; Demande US 2014134310). Ces produits de dépolymérisation de la pectine sont constitués d'au moins deux unités α-1,4-D-galacturonate. Il s'agit également de tout produit de dépolymérisation de la pectine ayant subi une hydrolyse par voie chimique et/ou physique (Demande internationale WO 2009/004153).

Par « ulvanes » au sens de la présente invention, on entend des polysaccharides anioniques sulfatés solubles dans l'eau, extraits d'algues vertes de type ulve ou entéromorphe, par exemple issus des espèces *Ulva armoricana, Ulva rigida, Ulva rotundata, Ulva lactuca, Ulvan linza, Enteromorpha intestinalis* et *Entoromorpha compressa,* par exemple obtenus selon le protocole décrit dans le brevet EP1729575.

Par « oligoulvanes » au sens de la présente invention, on entend les oligosaccharides dérivés d'ulvanes obtenus par hydrolyse acide ou enzymatique, par exemple selon les protocoles décrits dans le brevet EP 2582810. Ces produits de dépolymérisation de l'ulvane sont constitués d'au moins deux unités acide D-glucuronique et acide L-iduronique.

Dans cette forme d'exécution où les acides uroniques sont constitués d'un hydrolysat d'oligo- ou polysaccharides comprenant un ou plusieurs motifs identiques ou différents d'acide uronique éventuellement salifiés, le procédé selon l'invention comprend en outre une étape d'hydrolyse acide permettant de transformer les poly- et oligosaccharides précédemment décrits en acides uroniques. Dans un premier mode de réalisation de l'invention, dit "mode combiné" et qui sera décrit en détail ci-après, l'étape d'hydrolyse est réalisée simultanément à l'étape de réaction avec l'alcool. Dans un second mode de réalisation, dit "mode séparé" et qui sera décrit en détail ci-après, l'étape d'hydrolyse est réalisée avant l'étape de réaction avec l'alcool.

Dans tous les cas, l'hydrolyse est réalisée dans un solvant constitué d'eau, d'un solvant ionique, d'un solvant eutectique ou de leurs mélanges, de préférence dans l'eau, au moyen d'au moins un acide tel que par exemple l'acide chlorhydrique ; l'acide sulfurique ; un acide alkyl sulfurique tel que l'acide décyl ou lauryl sulfurique ; un acide arylsulfonique tel que l'acide benzènesulfonique, l'acide paratoluène sulfonique ; l'acide camphosulfonique ; un acide alkylsulfonique tel que l'acide méthanesulfonique (ou méthylsulfonique), l'acide décylsulfonique, l'acide laurylsulfonique ; l'acide sulfosuccinique ou un sulfosuccinate d'alkyle tel le sulfosuccinate de décyle ou de lauryle ; les acides perhalohydriques, tel que l'acide perchlorique ; et leurs mélanges. De préférence, il s'agit d'acide alkylsulfonique tel que l'acide méthanesulfonique. En variante encore, le catalyseur acide peut être un catalyseur hétérogène tel que par exemple des zéolites (notamment une beta zéolite), des résines échangeuses d'ions (telles que les résines Amberlyst et Nafion), des silices mésoporeuses fonctionnalisées, des carbones fonctionnalisés, des oxydes métalliques supportés et fonctionnalisés, des hétéropolyacides, et leurs mélanges, de préférence une résine échangeuse de cations à groupements acides forts, notamment acide sulfonique, greffés sur du polystyrène ou un copolymère de styrène éventuellement réticulé par du divinylbenzène, telle que la résine Amberlyst-15.

Dans le second mode de réalisation de l'invention, dit "mode séparé", on peut mettre en présence 1 équivalent de poly- ou oligosaccharides ; de 2 à 700 équivalents molaires d'eau, de préférence entre 50 et 400 équivalents molaires ; et de 10⁻³ à 10 équivalents molaires de catalyseur acide et de préférence de 1,5 à 4,5 équivalents molaires d'acide alkylsulfonique. Cette étape d'hydrolyse est préférentiellement conduite entre 80 °C et 130 °C, et de préférence portée au reflux de l'eau à la pression atmosphérique sur un temps de réaction pouvant être de 1 à 24 heures et de préférence sur un temps de réaction de 5 à 8 heures.

Dans une autre forme d'exécution de l'invention, on utilise des acides uroniques monomères, de sorte que l'étape d'hydrolyse précitée n'est pas nécessaire. Par « acides uroniques monomères » au sens de la présente invention, on entend des monosaccharides issus de la dépolymérisation de polysaccharides naturels contenant des sucres uroniques pour accéder à des unités monomériques ou bien à des acides uroniques obtenus par tout autre procédé (par exemple l'oxydation d'hexoses en acides uroniques, comme l'oxydation du D-glucose en acide D-glucuronique), ainsi que leurs esters, notamment sous la forme de lactone. L'acide uronique peut par exemple être choisi parmi l'acide guluronique, l'acide glucuronique, l'acide galacturonique, l'acide mannuronique, l'acide iduronique, la glucurono-6,3-lactone et leurs mélanges.

Dans le procédé selon l'invention, les acides uroniques sont transformés par réaction avec au moins un alcool de formule R-OH où R désigne un groupe alkyle ou alcényle en C₁-C₂₂, en présence d'au moins un catalyseur acide. Cette étape de transformation implique en principe des réactions d'estérification, de glycosidation, de contraction de cycle et de déshydratation.

Dans le cas où on utilise un hydrolysat d'oligo- ou polysaccharides, l'alcool mis en œuvre dans l'étape de transformation précitée est de formule R-OH où R désigne un groupe alkyle ou alcényle en C₁-C₆. L'utilisation d'alcools à chaîne alkyle plus longue peut en effet affecter le rendement du procédé. En revanche, si des acides uroniques monomères sont mis en œuvre dans le procédé selon l'invention, l'étape de transformation de ces acides uroniques peut être effectuée directement avec au moins un alcool de formule R-OH où R désigne un groupe alkyle ou alcényle en C₁-C₂₂.

Des exemples d'alcools utilisables dans le procédé selon l'invention sont les alcools linéaires ou ramifiés, saturés ou insaturés, tels que l'éthanol, le propanol, l'isopropanol, le butanol, l'isobutanol, l'hexanol, l'octanol, le 2-éthyl-hexanol, le décanol, l'alcool laurylique, l'alcool myristylique, l'alcool cétylique, l'alcool oléylique, l'alcool stéarylique, l'alcool cétéarylique, l'alcool arachidonylique, et leurs mélanges, sans que cette liste ne soit limitative. Il est bien entendu que, dans le cas où un seul alcool est mis en œuvre, les substituants R₁, R2 et R3 de la formule (I) seront identiques, tandis qu'ils peuvent être différents les uns des autres en présence de plusieurs alcools R-OH.

L'étape de transformation est réalisée en présence non seulement d'un alcool mais également d'un catalyseur acide qui peut être un catalyseur homogène tel qu'un acide de Brønsted choisi parmi les acides cités précédemment, ou un acide de Lewis, choisi par exemple parmi les halogénures de cuivre, d'argent, de manganèse, de fer, de ruthénium, de magnésium ou d'aluminium ou les composés de formule ZnR2, SnR2, SnR4 et SiR4 où les groupements R désignent, indépendamment les uns des autres, un atome d'halogène ou un groupe alkyle, cycloalkyle, alcényle, phényle ou benzyle. En variante encore, le catalyseur acide peut être un catalyseur hétérogène tel que par exemple des zéolites (notamment une beta zéolite), des résines échangeuses d'ions (telles que les résines Amberlyst et Nafion), des silices mésoporeuses fonctionnalisées, des carbones fonctionnalisés, des oxydes métalliques supportés et fonctionnalisés, des hétéropolyacides, et leurs mélanges, de préférence une résine échangeuse de cations à groupements acides forts, notamment acide sulfonique, greffés sur du polystyrène ou un copolymère de styrène éventuellement réticulé par du divinylbenzène, telle que la résine Amberlyst-15.

Dans les modes de réalisation "combiné" et "séparé" de l'invention, l'étape de transformation est généralement réalisée en présence du catalyseur acide utilisé dans l'étape d'hydrolyse.

Dans une variante du mode de réalisation "séparé" de l'invention, l'étape d'hydrolyse est suivie d'une étape de neutralisation puis d'élimination de l'eau, lesdites étapes étant réalisées successivement avant l'étape de transformation. Dans cette variante, le catalyseur acide utilisé dans l'étape de transformation peut être identique à l'acide utilisé dans l'étape d'hydrolyse ou différent de celui-ci.

Dans le mode de réalisation "combiné" de l'invention, on peut par exemple mettre en présence 1 équivalent d'oligo- ou polysaccharides ; de 2 à 500 équivalents molaire d'eau, de préférence 5 à 50 équivalents molaires dans le cas d'oligosaccharides et 50 à 150 équivalents molaires dans le cas de polysaccharides ; de 2 à 300 équivalents molaires d'alcool, de préférence de 50 à 100 équivalents molaires dans le cas d'oligosaccharides et de 150 à 180 équivalents molaires dans le cas de polysaccharides ; de 1 à 10 équivalents molaires de catalyseur acide, de préférence de 4 à 8 équivalents molaires d'acide alkylsulfonique.

Dans le cas où on utilise des monosaccharides comme matières premières (absence d'étape d'hydrolyse), les conditions de l'étape de transformation peuvent être légèrement ajustées. Par exemple, l'étape de transformation peut être réalisée en mettant en présence 1 équivalent d'acide D-glucuronique, d'acide D-galacturonique ou de D-glucurono-6,3-lactone, de 2 à 200 équivalents molaires d'alcool et de 1 à 50 équivalents molaires de catalyseur acide, de préférence de 1 à 5 équivalents molaires d'acide alkylsulfonique. La réaction peut être menée sur 1 à 6 heures. Il n'est pas nécessaire d'effectuer cette étape en présence d'un solvant, tel que de l'eau.

La réaction de transformation est généralement conduite à pression atmosphérique. Pour obtenir les composés de formule (I), le milieu réactionnel est nécessairement porté à une température de 150 à 180°C, de préférence de 155 à 170°C. Pour ce faire, on préfère que le milieu réactionnel soit porté à 150-180°C dès le début de la réaction. La réaction est par exemple conduite sur une période de 6 à 24 heures. En variante, le milieu réactionnel peut être porté à 150-180°C après que la réaction ait été amorcée à 80-140°C pendant 6 à 12h.

Dans le mode de réalisation "séparé" de l'invention, et dans le cas où une étape de neutralisation est prévue avant l'étape de transformation, le milieu issu de l'étape d'hydrolyse est généralement mis en présence d'une solution aqueuse contenant une base M(OH)ₓ dans laquelle M est un métal alcalin ou alcalino-terreux, et x est la valence. Cette étape est réalisée, par exemple, en introduisant dans le milieu réactionnel issu de l'étape d'hydrolyse, une fois ramené à température ambiante, de 10⁻³ à 10 équivalents molaires d'une solution aqueuse contenant une base de formule M(OH)ₓ telle que définie ci-dessus, et de préférence de 1 à 3 équivalents molaires d'une solution d'hydroxyde de sodium 1N. Le milieu peut ensuite être concentré par élimination totale ou partielle de l'eau (par exemple par distillation sous pression réduite ou par lyophilisation). L'étape de transformation est alors réalisée, par exemple, en introduisant dans le milieu neutralisé et éventuellement concentré issu de l'étape de neutralisation ci-dessus de 2 à 300 équivalents molaires d'alcool, de préférence de 100 à 200 équivalents molaires d'alcool, et de 1 à 10 équivalents molaires de catalyseur acide, de préférence de 5 à 10 équivalents molaires d'acide alkylsulfonique.

L'étape de neutralisation précitée peut être utile dans le cas où l'on souhaite stocker le produit intermédiaire formé mais elle n'est pas indispensable. En l'absence d'une telle étape, le milieu issu de l'étape d'hydrolyse est directement engagé dans l'étape de transformation, par exemple en introduisant dans le milieu réactionnel issu de l'étape d'hydrolyse 2 à 300 équivalents molaires d'alcool et de préférence de 100 à 200 équivalents molaires d'alcool. Il n'est pas nécessaire d'ajouter un catalyseur dans ce cas.

Quel que soit le mode de réalisation mis en œuvre, on obtient à l'issue du procédé décrit précédemment un produit renfermant d'environ 25 à 45% en poids du composé de formule (I).

Dans le cas où le procédé a été mis en œuvre sur un hydrolysat d'oligo- ou polysaccharides, initialement engagé dans l'étape de transformation avec un alcool de formule R-OH où R désigne un groupe alkyle ou alcényle en C₁-C₆, et où l'on souhaite obtenir des 5-dialkylacétal-2-furanoates d'alkyle présentant des chaînes alkyles plus longues, le procédé comprend alors éventuellement en outre une étape de transestérification et transacétalisation du composé de formule (I) ainsi obtenu avec un alcool R'-OH où R' représente un groupe alkyle ou alcényle en C₇-C₂₂, de préférence en C₈-C₁₈ et, mieux, en C₁₂-C₁₈, tel que le dodécanol ou l'alcool oléique. Du fait que les groupes R₁, R₂, R₃ des composés de formule (I) ne sont pas nécessairement identiques, que la réaction de transestérification n'est pas nécessairement totale et qu'un mélange d'alcools R'-OH peut être mis en jeu, les trois groupes alkyles des 5-dialkylacétal-5-furanoates d'alkyle obtenus à l'issue de cette réaction pourront être différents les uns des autres, ou l'un des groupes alkyles pourra être différent des deux autres. Il est toutefois possible que chacun des groupes R₁, R₂ et R₃ soit remplacé par le même groupe R'.

Cette étape peut être effectuée de manière classique. Par exemple, le milieu réactionnel issu de l'étape de transformation peut être d'abord soumis à une étape de neutralisation partielle en présence d'eau et d'une base M(OH)ₓ dans laquelle M est un métal alcalin ou alcalino-terreux, et x est la valence. Cette étape est réalisée, par exemple, en introduisant dans le milieu réactionnel, une fois ramené à température ambiante, de 0,1 à 10 équivalents molaires d'une solution aqueuse contenant une base de formule M(OH)ₓ telle que définie ci-dessus, et de préférence de 1 à 3 équivalents molaires d'une solution d'hydroxyde de sodium 1N. L'étape de transestérification et transacétalisation peut être réalisée, par exemple, en introduisant dans le milieu réactionnel partiellement neutralisé, de 2 à 50 équivalents molaires d'alcool de formule R'-OH tel que défini ci-dessus, et de préférence de 5 à 15 équivalents molaires. Les réactions de transestérification et transacétalisation sont ensuite par exemple poursuivies en permettant de recycler l'alcool à chaine courte préalablement utilisé pour la formation du 5-dialkylacétal-2-furoate d'alkyle en présence de l'eau provenant de la solution aqueuse contenant la base de formule M(OH)ₓ. La réaction peut notamment être conduite sur une durée de 1 heure à 24 heures, de préférence pendant 3 à 5 heures, à une température comprise entre 50 et 160°C, de préférence entre 65 et 85°C et avantageusement sous pression réduite pour le recyclage de l'alcool précédemment cité et de l'eau.

Le produit susceptible d'être obtenu suivant ce procédé est caractérisé en ce qu'il renferme le composé de formule (I) associé à un furoate d'alkyle.

Précisément, outre le composé de formule (I) ou son produit de transestérification et transacétalisation, le produit issu du procédé selon l'invention renferme une faible quantité de furoate d'alkyle (entre 2 et 5 % massique). Ce produit peut en outre contenir au plus 10 % massique d'alkylosides uronates d'alkyle (alkylguloside/mannoside/galactoside/glucoside/idoside uronates d'alkyle).

Dans la mesure où ce dernier est utile pour plusieurs applications, notamment en tant que solvant, agent de coalescence, inhibiteur de cristallisation, agent plastifiant, agent de dégraissage, agent décapant, agent nettoyant ou agent d'augmentation de l'activité biologique (brevet FR3002535), le procédé selon l'invention peut comprendre une étape consistant à isoler le furoate d'alkyle co-produit avec le composé de formule (I). A défaut, ce dernier peut être simplement éliminé par distillation sous pression réduite.

Pour isoler les dérivés furanoïques décrits précédemment, l'étape d'estérification ou de transestérification du procédé "combiné" ou du procédé "séparé" est suivie d'une étape de neutralisation du milieu réactionnel, en présence d'une solution aqueuse contenant une base M(OH)x dans laquelle M est un métal alcalin ou alcalino-terreux, et x est la valence. Cette étape est réalisée, par exemple, en introduisant dans le milieu réactionnel, une fois ramené à température ambiante, de 10⁻³ à 10 équivalents molaires d'une solution aqueuse contenant une base de formule M(OH)ₓ telle que définie ci-dessus, et de préférence de 1 à 3 équivalents molaires d'une solution d'hydroxyde de sodium 1N. Le milieu est ensuite généralement concentré par élimination totale de l'eau, par exemple par distillation sous pression réduite ou par lyophilisation. Le résidu peut ensuite être repris dans un solvant polaire tel que l'éther diéthylique et éventuellement soumis à des ultrasons afin d'obtenir une poudre fine. La suspension ainsi obtenue peut être filtrée et le filtrat peut ensuite être concentré sous pression réduite. Une distillation sous pression réduite (par exemple par distillation moléculaire) permet de récupérer le furoate d'alkyle dans le distillat et le composé de formule (I) dans le résidu.

Le procédé selon l'invention peut être poursuivi pour préparer des sels d'acide 5-dialkylacétal-2-furoïque de formule (II) : 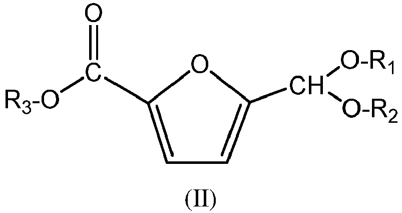 dans laquelle : R₁ et R₂ désignent chacun indépendamment un groupe alkyle ou alcényle en C₁-C22 ; et R₃ désigne un atome de métal alcalin ou de métal alcalino-terreux ou un groupe ammonium quaternaire de formule (III) :

-N⁺-R₄R₅R₆R₇ (III)

où R₄, R₅, R₆ et R₇ désignent chacun indépendamment un atome d'hydrogène, un groupe alkyle en C₁-C₆ ou un groupe hydroxyalkyle en C₁-C₆.

Pour ce faire, le procédé comprend en outre une étape de saponification du produit de formule (I) ou du produit de transestérification et transacétalisation précité. Il n'est pas nécessaire d'isoler le composé de formule (I) avant de réaliser cette saponification. Cette réaction peut être effectuée en présence (i) d'une base M(OH)ₓ dans laquelle M est un métal alcalin ou alcalino-terreux, et x est la valence, ou (ii) d'une base 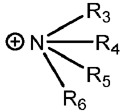 où chacun des R₃ à R₆ est indépendamment un atome d'hydrogène, un alkyle ayant de 1 à 6 atomes de carbone ou un hydroxyalkyle ayant de 1 à 6 atomes de carbone. Cette étape de saponification de l'ester est réalisée, par exemple, en introduisant dans le milieu réactionnel, de 0,5 à 10 équivalents, et de préférence 2 à 5 équivalents, d'une base telle que définie ci-dessus. Avantageusement, la base est l'hydroxyde de sodium, l'hydroxyde de potassium, l'ammoniaque ou un hydroxyde d'alkyl (hydroxyalkyl) ammonium. La réaction de saponification des esters est préférentiellement conduite à une température comprise entre 0 °C et 150 °C, et de préférence entre 100 et 120°C, pendant une durée de 15 min à 24 heures, et de préférence pendant 6 à 15 heures.

Les alcools présents à l'issue de l'étape de saponification sont avantageusement éliminés pour récupérer les sels précités. Les alcools en C₁-C₁₀ présents à l'issue de l'étape de saponification peuvent être éliminés par distillation azéotropique avec l'eau. Les alcools en C₁₀-C₂₂ présents à l'issue de l'étape de saponification peuvent être éliminés partiellement ou totalement par distillation moléculaire ou par extraction solide-liquide avec un solvant organique, de préférence par extraction solide-liquide avec de l'acétone.

Les sels précités peuvent éventuellement être acidifiés à l'issue de l'étape de saponification (avec ou sans élimination préalable des alcools) pour obtenir des acides 5-dialkylacétal-2-furoïques. Cette réaction peut par exemple être effectuée en présence d'un acide choisi dans le groupe constitué de : l'acide chlorhydrique, l'acide sulfurique, l'acide oxalique, un acide sulfonique ou une résine sulfonique sous sa forme H⁺. Les sels inorganiques peuvent être éliminés par extraction liquide-liquide. Cette réaction d'acidification est réalisée, par exemple, en mettant en présence 1 équivalent de sels d'acide 5-dialkylacétal-2-furoïque avec 1 équivalent ou plus d'un acide tel que défini ci-dessus. Après une éventuelle purification par chromatographie sur gel de silice, l'acide 5-dialkylacétal-2-furoïque est obtenu.

A partir de l'acide 5-dialkylacétal-2-furoïque, il est possible de retrouver sa forme de sel en le dispersant dans l'eau et en ajoutant par exemple de 0,5 à 10 équivalents, et de préférence de 1 à 1,5 équivalent, d'une base. De préférence, la base est l'hydroxyde de sodium, l'hydroxyde de potassium, l'ammoniaque ou un hydroxyde d'alkyl (hydroxyalkyl) ammonium. La réaction peut être effectuée sous agitation à une température comprise entre 50 et 100°C, de préférence entre 70 et 90°C, sur une période de 1 à 12 heures, et de préférence pendant 2 à 4 heures. Le milieu peut ensuite être refroidi à température ambiante puis l'eau éliminée par distillation sous pression réduite ou par lyophilisation.

Les sels d'acide 5-dialkylacétal-2-furoïque ont des propriétés tensioactives et peuvent donc être utilisés comme agents tensioactifs, notamment dans des compositions détergentes. Par "agent tensioactif", on entend aussi bien des agents solubilisants, hydrotropes, mouillants, moussants, émulsionnants, émulsifiants et/ou détergents. Les sels d'acide 5-dialkylacétal-2-furoïque selon l'invention présentent en particulier les propriétés suivantes :

| Nombre d'atomes de carbone des 2 chaînes lipophiles (dialkylacétal) : | Sels d'acide 5-dialkylacétal-2-furoïque |
|---|---|
| Entre 1 et 6 | Agents hydrotropes et/ou solubilisants |
| Entre 6 et 14 | Agents moussants et/ou détergents |
| Entre 16 et 22 | Agents émulsionnants |

Une tension superficielle de 32 (± 2) mN/m peut être obtenue pour une concentration en sel d'acide 5-dialkylacétal-2-furoïque inférieure ou égale à 0,8 g/L, de préférence inférieure ou égale à 0,6 g/L, mieux encore inférieure ou égale à 0,4 g/L.

Ces sels peuvent en particulier être utilisés dans la préparation de détergents ménagers, hospitaliers et/ou industriels, notamment pour le lavage du linge et de la vaisselle ; de détergents cosmétiques, notamment de shampooings, de dentifrices ou de compositions de nettoyage de la peau, tels que des gels douche ; de compositions cosmétiques non rincées, telles que des crèmes de soin de la peau ; de compositions destinées au forage ou au raffinage du pétrole ; de peintures ; de produits phytosanitaires ; de compositions de traitement des bitumes ; de compositions de traitement des textiles, notamment d'ensimage ou de teinture ; de compositions utilisées dans l'industrie métallurgique.

L'invention a également pour objet une composition détergente renfermant ces sels, préférentiellement un détergent ménager ou cosmétique.

### EXEMPLES

L'invention sera mieux comprise à la lumière des exemples suivants, qui sont donnés à titre purement illustratif et n'ont pas pour but de limiter la portée de l'invention, définie par les revendications annexées.

### A- Exemples de procédés de synthèse selon l'invention

### Exemple 1 : Synthèse du 5-dibutylacétal-2-furoate de butyle à partir d'oligomannuronate et de butanol

L'oligomannuronate a été obtenu selon le procédé décrit dans la Demande Internationale WO 201709817 et présentait les propriétés suivantes :

| **Libellé** | **Méthode** | **Résultat** | **Unités** |
|---|---|---|---|
| **Matière sèche** | Masse constante à 103°C | 95,6 | %sec/brut |
| **Matière minérale** | Four à mouffles, 550°C | 31,9 | %sec/brut |
| **Rapport (M/G)** | Par calcul, méthanolyse | 2,7 | |
| **DP** | Par calcul | 4,0 | |

L'oligomannuronate (1.0 g, 3.62 mmol CO₂⁻, 1,0 éq) a été dispersé dans l'eau (0.85 mL, 47.1 mmol, 13.0 éq) et le butanol (25 mL, 275 mmol, 76.0 éq). Une solution d'acide méthanesulfonique 70% (2.21 g, 16.1 mmol, 4.45 éq) a été ajoutée et le mélange a été chauffé à 160°C. L'eau formée dans le milieu a progressivement été éliminée par distillation azéotropique. Après 6 heures de réaction, le mélange a été refroidi à température ambiante. Le milieu réactionnel a ensuite été neutralisé avec une solution aqueuse de NaOH 1M (10,5 mL, pH 7-8) à température ambiante puis il a été concentré et séché sous pression réduite. Le résidu est repris dans de l'Et₂O et passé aux ultrasons afin d'obtenir une poudre fine. La suspension est ensuite filtrée sur célite puis lavée à l'Et₂O jusqu'à disparition du produit désiré dans le filtrat. Le filtrat est concentré sous pression réduite et séché sur la rampe à vide (m_{brut} = 917 mg). Une analyse par RMN ¹H montre la présence du 2-furoate de butyle à hauteur de 12% par rapport au 5-dibutylacétal-2-furoate de butyle. Le 2-furoate de butyle peut être récupéré par distillation sous pression réduite (température d'ébullition : 83-84°C, 1,00 mmHg). Après purification par chromatographie sur gel de silice (Cyclohexane + 1% Et3N), le 5-dibutylacétal-2-furoate de butyle a été isolé (389 mg) avec une pureté de l'ordre de 99% (rendement molaire = 33 %, rendement massique = 60%).

### Exemple 2 : Synthèse du 5-dibutylacétal-2-furoate de butyle à partir d'oligoguluronate et de butanol

L'oligoguluronate a été obtenu selon le procédé décrit dans la Demande Internationale WO 201709817 et présentait les propriétés suivantes :

| **Libellé** | **Méthode** | **Résultat** | **Unités** |
|---|---|---|---|
| **Matière sèche** | Masse constante à 103°C | 100,0 | %sec |
| **Matière minérale** | 12h, 550°C | 26,3 | %sec/brut |
| **Rapport (M/G)** | Par calcul, RMN du proton | 0,039 | |
| **DP** | Par calcul | 30,0 | %sec/brut |

L'oligoguluronate 2014-NVR-201 (1.0 g, 4.22 mmol CO2-, 1.0 éq) a été dispersé dans l'eau (0.99 mL, 54.9 mmol, 13.0 éq) et le butanol (29.4 mL, 321 mmol, 76.0 éq). Une solution d'acide méthanesulfonique 70% (2.58 g, 18.80 mmol, 4.45 éq) a été ajoutée et le mélange a été chauffé à 160°C. L'eau formée dans le milieu a progressivement été éliminée par distillation azéotropique. Après 10 heures de réaction, le mélange a été refroidi à température ambiante. Le milieu réactionnel a ensuite été neutralisé avec une solution aqueuse de NaOH 1M (14.6 mL, pH 7-8) à température ambiante puis il a été concentré et séché sous pression réduite. Le résidu est repris dans de l'Et₂O et passé aux ultrasons afin d'obtenir une poudre fine. La suspension est ensuite filtrée sur célite puis lavée à l'Et₂O jusqu'à disparition du produit désiré dans le filtrat. Le filtrat est concentré sous pression réduite et séché sur la rampe à vide (m_{Brut} = 865 mg).

Après purification par chromatographie sur gel de silice (Cyclohexane + 1% Et₃N), une analyse par RMN ¹H (CDCl₃, 400 MHz) du brut réactionnel a confirmé la présence d'un mélange de 2-furoate de butyle et de 5-dibutylacétal-2-furoate de butyle.

### Exemple 3 : Synthèse du 5-diisobutylacétal-2-furoate d'isobutyle à partir d'oligomannuronate et d'isobutanol

L'oligomannuronate obtenu comme décrit à l'Exemple 1 (1.0 g, 3.62 mmol CO₂⁻, 1,0 éq) a été dispersé dans l'eau (0.85 mL, 47.1 mmol, 13.0 éq) et du 2-butanol (25 mL, 275 mmol, 76.0 éq). Une solution d'acide méthanesulfonique 70% (2.21 g, 16.1 mmol, 4.45 éq) a été ajoutée et le mélange a été chauffé à 160°C. L'eau formée dans le milieu a progressivement été éliminée par distillation azéotropique. Après 6 heures de réaction, le mélange a été refroidi à température ambiante.

Le milieu réactionnel a ensuite été neutralisé avec une solution aqueuse de NaOH 1M (11 mL, pH 7-8) à température ambiante puis il a été concentré et séché sous pression réduite. Le résidu est repris dans de l'Et₂O et passé aux ultrasons afin d'obtenir une poudre fine. La suspension est ensuite filtrée sur célite puis lavée à l'Et₂O jusqu'à disparition du produit désiré dans le filtrat. Le filtrat est concentré sous pression réduite et séché sur la rampe à vide (m_{brut} = 809 mg).

Après purification par chromatographie sur gel de silice (Cyclohexane + 1% Et3N), le 5-diisobutylacétal-2-furoate d'isobutyle a été isolé (132 mg) avec une pureté de l'ordre de 99% (rendement molaire = 11 %, rendement massique = 20 %).

### Exemple 4 : Synthèse du 5-dihexylacétal-2-furoate d'hexyle à partir d'oligomannuronate et d'hexanol

L'oligomannuronate obtenu comme décrit à l'Exemple 1 (1.0 g, 3.62 mmol CO₂⁻, 1,0 éq) a été dispersé dans l'eau (0.85 mL, 47.1 mmol, 13.0 éq) et l'hexanol (34.5 mL, 275 mmol, 76.0 éq). Une solution d'acide méthanesulfonique 70% (2.21 g, 16.1 mmol, 4.45 éq) a été ajoutée et le mélange a été chauffé à 175°C. L'eau formée dans le milieu a progressivement été éliminée par distillation azéotropique. Après 6 heures de réaction, le mélange a été refroidi à température ambiante.

Le milieu réactionnel a ensuite été neutralisé avec une solution aqueuse de NaOH 1M (12.4 mL, pH 7-8) à température ambiante puis il a été concentré et séché sous pression réduite. Le résidu est repris dans de l'Et₂O et passé aux ultrasons afin d'obtenir une poudre fine. La suspension est ensuite filtrée sur célite puis lavée à l'Et₂O jusqu'à disparition du produit désiré dans le filtrat. Le filtrat est concentré sous pression réduite et séché sur la rampe à vide (m_{brut} = 1376 mg).

Après purification par chromatographie sur gel de silice (Cyclohexane + 1 % Et3N), un mélange a été obtenu (189 mg) contenant le 5-dihexylacétal-2-furoate d'hexyle à 85% (rendement molaire = 11 %, rendement massique = 25 %) ainsi que le 2-furoate d'hexyle à 15 %.

### Exemple 5 : Synthèse du 5-dibutylacétal-2-furoate de butyle à partir d'alginate et de butanol

Un alginate raffiné issu de *Laminaria digitata* a été obtenu selon le procédé décrit dans la Demande Internationale WO 201709817. L'alginate (1.0 g, 3.38 mmol CO₂⁻, 1.0 éq) a été dispersé dans le butanol (53.2 mL, 581.36 mmol, 172 éq) et dans l'eau (6.1 mL, 338 mmol, 100 éq) et une solution d'acide méthanesulfonique 70% (2.78 g, 20.28 mmol, 6.0 éq) a été ajoutée. Le mélange a été chauffé à 110°C sous vive agitation. Après 8 heures de réaction, le milieu réactionnel est laissé revenir à température ambiante puis un Dean-Stark est ajouté au montage afin d'éliminer l'eau par distillation azéotropique. Le mélange est chauffé à 160°C. Après 15 heures de réaction supplémentaires, le mélange est laissé revenir à température ambiante.

Le milieu réactionnel a ensuite été neutralisé avec une solution aqueuse de NaOH 1M (15.0 mL, pH 7-8) à température ambiante puis il a été concentré et séché sous pression réduite. Le résidu est repris dans de l'Et₂O et passé aux ultrasons afin d'obtenir une poudre fine. La suspension est ensuite filtrée sur célite puis lavée à l'Et₂O jusqu'à disparition du produit désiré dans le filtrat. Le filtrat est concentré sous pression réduite et séché sur la rampe à vide (m_{brut} = 739 mg).

Après purification par chromatographie sur gel de silice (Cyclohexane + 1% Et₃N), 304 mg de 5-dibutylacétal-2-furoate de butyle a été isolé avec une pureté de l'ordre de 99% (rendement molaire = 30 %, rendement massique = 52%).

### Exemple 6 : Synthèse du 5-dibutylacétal-2-furoate de butyle à partir d'ulvane et de butanol

Des ulvanes ont été obtenus selon le procédé décrit dans la Demande Internationale WO 201709817 et présentaient les propriétés suivantes :

| **Libellé** | **Méthode** | **Résultat** | **Unités** |
|---|---|---|---|
| **Matière sèche** | Masse constante à 103°C | 87.3 | %sec/brut |
| **Matière minérale** | 12 h, 550°C | 14.7 | %sec/brut |
| **Teneur en sulfates** | Méthode turbidimétrique | 19.3 | %/sec |
| **Teneur en L-(+)-Rhamnose** | Méthanolyse | 23.1 | %/sec |
| **Teneur en acide iduronique** | Méthanolyse | 5.3 | %/sec |
| **Teneur en acide (D)-galacturonique** | Méthanolyse | <0.1 | %/sec |
| **Teneur en acide (D)-glucuronique** | Méthanolyse | 12.9 | %/sec |
| **Masse moléculaire (éq pullulan)** | Exclusion stérique, Colonne « haute masse » | 1.04 x 10⁶ | %/sec |

Les ulvanes (500 mg, 2.097 mmol CO₂⁻, 1.0 éq) ont été dispersés dans le butanol (33.0 mL, 360.7 mmol, 172.0 éq) et dans l'eau (3.78 mL, 209.7 mmol, 100 éq) et une solution d'acide méthanesulfonique 70% (1.728 g, 12.58 mmol, 6.0 éq) a été ajoutée. Le mélange a été chauffé à 110°C sous vive agitation. Après 8 heures de réaction, le milieu réactionnel est laissé revenir à température ambiante puis le Dean-Stark est ajouté au montage afin d'éliminer l'eau par distillation azéotropique. Le mélange est chauffé à 160°C. Après 15 heures de réaction supplémentaires, le mélange est laissé revenir à température ambiante.

Le milieu réactionnel a ensuite été neutralisé avec une solution aqueuse de NaOH 1M (12.5 mL, pH 7-8) à température ambiante puis il a été concentré et séché sous pression réduite. Le résidu est repris dans de l'Et₂O et passé aux ultrasons afin d'obtenir une poudre fine. La suspension est ensuite filtrée sur célite puis lavée à l'Et₂O jusqu'à disparition du produit désiré dans le filtrat. Le filtrat est concentré sous pression réduite et séché sur la rampe à vide (m_{brut} = 268 mg).

Après purification par chromatographie sur gel de silice (Cyclohexane + 1 % Et₃N) 54 mg de 5-dibutylacétal-2-furoate de butyle ont été isolé avec une pureté de l'ordre de 95 % (rendement molaire = 23 %, rendement massique = 33 %).

### Exemple 7 : Synthèse du 5-dibutylacétal-2-furoate de butyle à partir d'oligomannuronate et de butanol

Un oligomannuronate a été préparé comme décrit dans l'Exemple 1. L'oligomannuronate (500 mg, 1.81 mmol CO₂⁻, 1,0 éq) a été dispersé dans l'eau (5 mL, 277.5 mmol, 153 éq). Une solution d'acide méthanesulfonique 70% (0.37 g, 2.7 mmol, 1.5 éq) a été ajoutée et le mélange a été chauffé à 110°C pendant 5 heures. Le milieu réactionnel a ensuite été neutralisé avec une solution aqueuse de NaOH 1M à température ambiante puis il a été concentré sous pression réduite. Du butanol (25 mL, 273.3 mmol, 151 éq) est additionné à l'hydrolysat puis de l'acide méthanesulfonique 70% (1.49 g, 10.86 mmol, 6 éq). Le milieu réactionnel a été agité à pression atmosphérique à 160°C pendant 12 heures.

Le milieu réactionnel a été refroidi à température ambiante, il a ensuite été neutralisé avec une solution aqueuse de NaOH 1M (pH 7-8) puis il a été concentré et séché sous pression réduite. Le résidu est repris dans de l'Et₂O et passé aux ultrasons afin d'obtenir une poudre fine. La suspension est ensuite filtrée sur célite puis lavée à l'Et₂O jusqu'à disparition du produit désiré dans le filtrat. Le filtrat est concentré sous pression réduite et séché sur la rampe à vide (m_{brut} = 375 mg).

Après purification par chromatographie sur gel de silice (Cyclohexane + 1% Et3N), le 5-dibutylacétal-2-furoate de butyle a été isolé (135 mg) avec une pureté de l'ordre de 99% (rendement molaire = 23 %, rendement massique = 42%).

### Exemple 8 : Synthèse du 5-dibutylacétal-2-furoate de butyle à partir d'alginate et de butanol

Un alginate raffiné issu de *Laminaria digitata* a été obtenu selon le procédé décrit dans la Demande Internationale WO 201709817. L'alginate (1.0 g, 3.38 mmol CO₂⁻, 1.0 éq) a été dispersé dans l'eau (24 mL) et une solution d'acide méthanesulfonique 70% (2.07 g, 15.04 mmol, 4.45 éq) a été ajoutée. Le mélange a été chauffé à 110°C sous vive agitation. Après 8 heures de réaction, du butanol (46.7 mL, 510.4 mmol, 151 éq) a été ajouté et le mélange a été chauffé à 160°C. L'eau présente dans le milieu a été progressivement éliminée par distillation azéotropique à l'aide d'un Dean-Stark. Après 15 heures de réaction supplémentaires, le mélange est laissé revenir à température ambiante.

Le milieu réactionnel a ensuite été neutralisé avec une solution aqueuse de NaOH 1M (10.2 mL, pH 7-8) à température ambiante puis il a été concentré et séché sous pression réduite. Le résidu est repris dans de l'Et₂O et passé aux ultrasons afin d'obtenir une poudre fine. La suspension est ensuite filtrée sur célite puis lavée à l'Et₂O jusqu'à disparition du produit désiré dans le filtrat. Le filtrat est concentré sous pression réduite et séché sur la rampe à vide (m_{brut} = 489 mg).

Après purification par chromatographie sur gel de silice (Cyclohexane + 1 % Et3N), un mélange de produits a été obtenu (198 mg) dont la composition massique est : 5-dibutylacétal-2-furoate de butyle 99% (rendement molaire = 18 %, rendement massique = 33%) ; 2-furoate de butyle <1%.

### Exemple 9 : Synthèse du 5-dibutylacétal-2-furoate de butyle à partir d'acide D-glucuronique et de butanol

L'acide D-glucuronique (300 mg, 1.55 mmol, 1.0 éq) a été dispersé dans du butanol (21 mL, 233 mmol, 151 éq) et de l'acide méthanesulfonique 99% (148.6 mg, 1.55 mmol, 1.0 éq) a été ajouté puis le mélange a été chauffé à 160°C sous vive agitation. L'eau présente dans le milieu a été progressivement éliminée par distillation azéotropique à l'aide d'un Dean-Stark Après 4h30 de réaction, le milieu réactionnel est laissé revenir à température ambiante.

Le milieu réactionnel a ensuite été neutralisé avec une solution aqueuse de NaOH 1M (1.6 mL, pH 7) à température ambiante puis il a été concentré sous pression réduite. Le résidu est repris dans de l'Et₂O et passé aux ultrasons afin d'obtenir une poudre fine. La suspension est ensuite filtrée sur célite puis lavée à l'Et₂O jusqu'à disparition du produit désiré dans le filtrat. Le filtrat est concentré sous pression réduite et séché sur la rampe à vide (m_{brut} = 358 mg).

Après purification par chromatographie sur gel de silice (Cyclohexane + 1% Et3N), le 5-dibutylacétal-2-furoate de butyle (160 mg) a été isolé avec une pureté supérieure à 95% (rendement molaire = 32 %, rendement massique = 51 %).

### Exemple 10 : Synthèse du 5-dibutylacétal-2-furoate de butyle à partir de D-Glucurono-6,3-lactone et de butanol

La D-Glucurono-6,3-lactone (500 mg, 2.84 mmol, 1.0 éq) a été dispersée dans du butanol (20.3 mL, 221.4 mmol, 78.0 éq) et de l'acide méthanesulfonique 99% (1.21 g, 12.6 mmol, 4.45 éq) a été ajouté puis le mélange a été chauffé à 160°C sous vive agitation. L'eau présente dans le milieu a été progressivement éliminée par distillation azéotropique à l'aide d'un Dean-Stark. Après 4h30 de réaction, le milieu réactionnel est laissé revenir à température ambiante.

Le milieu réactionnel a ensuite été neutralisé avec une solution aqueuse de NaOH 1M (11.0 mL, pH 7) à température ambiante puis il a été concentré sous pression réduite. Le résidu est repris dans de l'Et₂O et passé aux ultrasons afin d'obtenir une poudre fine. La suspension est ensuite filtrée sur célite puis lavée à l'Et₂O jusqu'à disparition du produit désiré dans le filtrat. Le filtrat est concentré sous pression réduite et séché sur la rampe à vide (m_{brut} = 669 mg).

Après purification par chromatographie sur gel de silice (Cyclohexane + 1% Et3N), le 5-dibutylacétal-2-furoate de butyle (233 mg) a été isolé avec une pureté supérieure à 95% (rendement molaire = 25 %, rendement massique = 47 %).

### Exemple 11: Synthèse du 5-dioctylacétal-2-furoate d'octyle à partir d'acide D-glucuronique et d'octanol

L'acide glucuronique (300 mg, 1.55 mmol, 1.0 éq) a été dispersé dans de l'octanol (36.6 mL, 233.5 mmol, 151 éq) et de l'acide méthanesulfonique 99% (148.6 mg, 1.55 mmol, 1.0 éq) a été ajouté, puis le mélange a été chauffé à 160°C sous vive agitation. Après 4h30 de réaction, le milieu réactionnel est laissé revenir à température ambiante.

Le milieu réactionnel a ensuite été neutralisé avec une solution aqueuse de NaOH 1M (2.2 mL, pH 7) à température ambiante puis il a été extrait dans l'acétate d'éthyle. La phase aqueuse a été extraite plusieurs fois à l'acétate d'éthyle puis les phases organiques regroupées sont lavées une fois à l'eau. La phase organique a ensuite été séchée sur Na₂SO₄, filtrée sur papier filtre puis concentrée sous pression réduite (m_{brut} = 1548 mg)

Après purification par chromatographie sur gel de silice (Cyclohexane + 1% Et3N), le 5-dioctylacétal-2-furoate d'octyle (136 mg) a été isolé avec une pureté supérieure à 90% (rendement molaire = 16 %, rendement massique = 41 %),. Le 2- furoate d'octyle (18 mg) a été isolé avec une pureté supérieure à 90%.

### Exemple 12: Synthèse de l'acide 5-dibutylacétal-2-furoïque et de son sel de sodium à partir d'oligomannuronate et de butanol

L'oligomannuronate obtenu comme décrit à l'Exemple 1 (4.0 g, 14.5 mmol CO₂⁻, 1,0 éq) a été dispersé dans l'eau (3.4 mL, 188.24 mmol, 13.0 éq) et le butanol (100.7 mL, 1100.5 mmol, 76.0 éq). La solution d'acide méthanesulfonique 70% (8.85 g, 64.4 mmol, 4.45 éq) a été ajoutée et le mélange a été chauffé à 160°C. L'eau formée dans le milieu a progressivement été éliminée par distillation azéotropique. Après 6 heures de réaction, le mélange a été refroidi à température ambiante.

A température ambiante, une solution aqueuse de NaOH 1M (72.7 mL, 72.7 mmol, 5.02 éq) a été ajoutée au milieu réactionnel sous vive agitation puis il a été chauffé à 110°C. Après 15 heures de réaction, le milieu réactionnel est laissé revenir à température ambiante.

A température ambiante et sous vive agitation, le milieu réactionnel est acidifié avec une solution aqueuse d'acide oxalique 0.5M (70 mL, 35.0 mmol, 2.42 éq, pH 2) puis il est extrait dans l'acétate d'éthyle. La phase aqueuse est extraite plusieurs fois dans l'acétate d'éthyle puis les phases organiques regroupées sont lavées une fois dans de l'eau. La phase organique est ensuite séchée sur Na₂SO₄, filtrée sur papier filtre puis le filtrat est concentré sous pression réduite (m_{Brut} = 3.211 g).

Après purification par chromatographie sur gel de silice avec un gradient d'élution Cyclohexane + 1% Et₃N/acétate d'éthyle, l'acide 5-dibutylacétal-2-furoïque (1089 mg) a été isolé avec une pureté supérieure à 95% (rendement molaire = 28 %, rendement massique = 42 %) ainsi qu'un mélange (388 mg) contenant de l'acide 2-furoïque ainsi que l'acide (*n*-butyl-α-D-mannopyranoside) uronique et l'acide (*n*-butyl-β-L-gulopyranoside) uronique.

A l'acide 5-dibutylacétal-2-furoïque (350 mg, 1.295 mmol, 1.0 éq) dispersé dans de l'eau (12.95 mL, C = 0.1 M) est ajoutée une solution aqueuse de NaOH 1M (1.55 mL, 1.55 mmol, 1.2 éq) puis le mélange est chauffé à 80°C. Après 3 heures de réaction, le milieu réactionnel limpide est laissé revenir à température ambiante puis l'eau est éliminée soit sous pression réduite soit par lyophilisation (m_{récupérée} = 371 mg, rendement molaire = 98 %).

### Exemple 13 : Synthèse d'acides 5-dialkylacétal-2-furoïque et de leurs sels de sodium à partir d'oligomannuronate et d'alcools gras

L'oligomannuronate obtenu comme décrit à l'Exemple 1 (500 mg, 1.81 mmol CO₂⁻, 1,0 éq) a été dispersé dans l'eau (0.42 mL, 23.5 mmol, 13.0 éq) et le butanol (12.6 mL, 137.6 mmol, 76.0 éq). La solution d'acide méthanesulfonique 70% (1.105 g, 8.05 mmol, 4.45 éq) a été ajoutée et le mélange a été chauffé à 160°C. L'eau formée dans le milieu a progressivement été éliminée par distillation azéotropique. Après 6 heures de réaction, le mélange a été refroidi à température ambiante.

A température ambiante, le milieu réactionnel est partiellement neutralisé avec une solution aqueuse de NaOH 1M (3.7 mL, 3.69 mmol, 2.04 éq) sous vive agitation puis le dodécanol (4 mL, 18.1 mmol, 10 éq) est ajouté au mélange. Le milieu réactionnel est chauffé à 75°C puis la pression est progressivement diminuée à 5 mbar de façon à éliminer tout le butanol résiduel. Après 4 heures de réaction dans ces conditions, le milieu réactionnel est laissé revenir à température ambiante et pression atmosphérique.

A température ambiante, une solution aqueuse de NaOH 0.4M (13.6 mL, 5.43 mmol, 3.0 éq) est ajoutée au milieu réactionnel puis il est chauffé à 110°C sous vive agitation. Après 15 heures de réaction, le milieu réactionnel est laissé revenir à température ambiante.

A température ambiante et sous vive agitation, le milieu réactionnel est acidifié avec une solution aqueuse d'acide oxalique 0.5M (12.0 mL, 6.0 mmol, 3.31 éq) puis il est extrait dans de l'acétate d'éthyle. La phase aqueuse est extraite plusieurs fois dans l'acétate d'éthyle puis les phases organiques regroupées sont lavées dans l'eau. La phase organique est ensuite séchée sur Na₂SO₄, filtrée sur papier filtre puis le filtrat est concentré sous pression réduite (m_{Brut} = 3.462 g).

Après purification par chromatographie sur gel de silice avec un gradient d'élution Cyclohexane + 1% Et₃N/Acétate d'éthyle, l'acide 5-didodécylacétal-2-furoïque (250 mg) a été isolé avec une pureté supérieure à 95% (rendement molaire = 28 %, rendement massique = 77 %).

**En suivant le même protocole mais en utilisant l'alcool oléique à la place du dodécanol** : rendement molaire = 31 %, rendement massique = 122 %.

**En suivant le même protocole mais en utilisant l'octanol à la place du dodécanol:** rendement molaire = 28%, rendement massique = 60%

A l'acide 5-dioctylacétal-2-furoïque (388 mg, 0.784 mmol, 1.0 éq) dispersé dans de l'eau (6.9 mL, C = 0.1 M) est ajoutée une solution aqueuse de NaOH 1M (0.94 mL, 0.94 mmol, 1.2 éq) puis le mélange est chauffé à 80°C. Après 3 heures de réaction, le milieu réactionnel limpide est laissé revenir à température ambiante puis l'eau est éliminée soit sous pression réduite soit par lyophilisation (m_{récupérée} = 401 mg, rendement molaire = 99 %).

**En suivant le même protocole mais en utilisant l'alcool oléique à la place du dodécanol:** rendement molaire = 98 %.

**En suivant le même protocole mais en utilisant l'octanol à la place du dodécanol:** rendement molaire = 100 %.

### Exemple 14 : Synthèse du 5-dioctylacétal-2-furoate de sodium à partir d'oligomannuronate et d'octanol

L'oligomannuronate obtenu comme décrit à l'Exemple 1 (2.0 g, 7.24 mmol CO₂⁻, 1,0 éq) a été dispersé dans l'eau (1.70 mL, 94.12 mmol, 13.0 éq) et le butanol (50.3 mL, 550.2 mmol, 76.0 éq). Une solution d'acide méthanesulfonique 70% (4.42 g, 32.2 mmol, 4.45 éq) a été ajoutée puis le mélange a été chauffé à 160 °C. L'eau formée dans le milieu a progressivement été éliminée par distillation azéotropique. Après 6 heures de réaction, le mélange a été refroidi à température ambiante.

A température ambiante, le milieu réactionnel est partiellement neutralisé avec une solution aqueuse de NaOH 1M (18.8 mL, 18.82 mmol, 2.6 éq) sous vive agitation puis l'octanol (5.66 g, 43.4 mmol, 6.0 éq) est ajouté au mélange. Le milieu réactionnel est chauffé à 75°C puis la pression est progressivement diminuée à 5 mbar de façon à éliminer tout le butanol résiduel. Après 4 heures de réaction dans ces conditions, le milieu réactionnel est laissé revenir à température ambiante et pression atmosphérique.

A température ambiante, une solution aqueuse de NaOH 0.4M (54.3 mL, 21.72 mmol, 3.0 éq) est ajoutée au milieu réactionnel puis il est chauffé à 110°C sous vive agitation. Après 15 heures de réaction, le milieu réactionnel est laissé revenir à température ambiante puis l'octanol est éliminé à 40 °C sous pression réduite par distillation azéotropique avec l'eau. Le résidu poudreux de couleur marron clair a été repris dans de l'eau distillée puis la suspension a été filtrée sur fritté afin d'éliminer tous les insolubles. Le filtrat a ensuite été lyophilisé et la présence du 5-dioctylacétal-2-furoate de sodium a été validée par RMN ¹H.

### Exemple 15 : Déprotection de la fonction aldéhyde du 5-dibutylacétal-2-furoate de butyle

Au 5-dibutylacétal-2-furoate de butyle obtenu à l'Exemple 1 (100 mg, 0.306 mmol, 1.0 éq) a été ajouté de l'acide para-toluène sulfonique monohydrate (86.2 mg, 0.453 mmol, 1.48 éq) puis le mélange a été placé sous atmosphère d'Argon. Il a ensuite été solubilisé dans du toluène anhydre (19 mL, C = 0.016 M) puis le milieu réactionnel a été chauffé au reflux sous atmosphère d'Argon.

Après 24 heures de réaction, le milieu réactionnel a été laissé revenir à température ambiante puis il a été neutralisé avec une solution aqueuse saturée de NaHCO₃ (15mL). Le milieu réactionnel a ensuite été extrait dans du dichlorométhane. La phase aqueuse a été extraite une fois supplémentaire dans du dichlorométhane. Les phases organiques regroupées ont ensuite été lavées avec une solution aqueuse saturée de NaCl. La phase organique a été séchée sur Na₂SO₄, filtrée sur papier filtre puis le filtrat a été concentré sous pression réduite (m_{Brut} = 66 mg).

Après purification par chromatographie sur gel de silice avec un gradient d'élution Cyclohexane/Acétate d'éthyle (95/5), le 5-formylfuran-2-furoate de butyle (30 mg) a été isolé avec une pureté supérieure 95 % (rendement molaire = 50 %, rendement massique = 30 %).

### B- Propriétés physico-chimiques des produits obtenus

### Méthodes

### - Solubilité

Une solution à 10 % massique de produit est préparée avec de l'eau MilliQ dans un flacon. Le mélange est agité manuellement par 3 allers-retours, puis il est laissé au repos à température ambiante.

La solubilisation du produit est jugée en fonction du temps mis par le produit pour être complètement dissout dans l'eau et former une solution homogène.

Si au bout de plusieurs jours, le mélange n'est toujours pas homogène (morceaux en suspension, solution laiteuse), il peut être alors chauffé à 40°C.

La température peut être augmentée au-dessus de 40°C si la dissolution n'est toujours pas effective.

### -pH

Le pH est mesuré sur une solution à 1 % massique de produit dans l'eau MilliQ.

### - CMC

La concentration micellaire critique (CMC) a été déterminée à l'aide de la méthode de l'anneau de Noüy ainsi que du logiciel « Krüss laboratory Desktop » avec le programme « Surfactant Characteristics ».

Une solution aqueuse (eau milliQ) du tensioactif a été préparée à différentes concentrations. 5 mL de cette solution sont utilisés pour la mesure de la CMC. L'eau milliQ est progressivement ajoutée à l'aide de la burette automatique contrôlée par le logiciel. La concentration et la tension superficielle sont automatiquement déterminées par le logiciel. La CMC a été mesurée à 25°C.

### - Pouvoir moussant

Une solution à 0,1% massique de produit est préparée avec de l'eau MilliQ à température ambiante. Un volume de 1,5 mL de la solution est prélevé et introduit dans un tube à hémolyse de 5 mL.

Le tube est bouché, puis agité manuellement par 3 allers-retours.

Le volume de mousse et la taille des bulles sont observés (photographie) juste après agitation, puis à 5 min et enfin à 30 min.

### - Tension interfaciale

La tension interfaciale (IFT) a été déterminée à l'aide de la méthode de l'anneau de Noüy ainsi que du logiciel « Krüss Laboratory Desktop » avec le programme « Surface and Interfacial Tension ». La méthode utilisée consistait à mesurer la force nécessaire pour tirer l'anneau à l'interface entre les deux phases (huile et eau).

Une solution huileuse (huile de tournesol) du tensioactif a été préparée à une concentration de 1 g/L. 35mL de cette solution huileuse sont introduits dans la cuve du tensiomètre. L'anneau de Noüy descend à la surface et mesure la tension de surface. La phase huileuse est ensuite remplacée par 35 mL d'eau milliQ. L'anneau de Noüy descend à la surface, détermine la tension de surface puis descend de quelques millimètres supplémentaires dans la phase aqueuse. La phase huileuse est ajoutée délicatement à la surface de la phase aqueuse avec une pipette en plastique. La tension interfaciale est alors déterminée automatiquement par le logiciel à 25°C.

Les propriétés physico-chimiques des sels d'acide 5-dialkylacétal-2-furanoïques FurCO₂Na C_{R'}C_{R'} ont été évaluées comme décrit ci-dessus et les différents résultats sont répertoriés dans le tableau ci-après.

| FurCO₂Na C_{R'} C_{R'} | 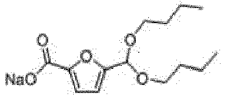 | 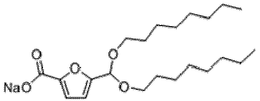 | 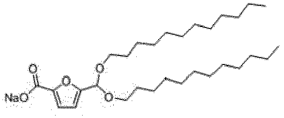 |
|---|---|---|---|
| Solubilisation | Immédiate | Soluble après 2 jours | Solution laiteuse après 2 jours à 40°C |
| pH | 11.53 | 10.60 | 10.12 |
| Tension interfaciale (mN/m) | 22.98 | 12.96 | 7.39 |
| Pouvoir moussant | Volume de mousse faibleBulles grossières Instable dans le temps | Volume de mousse important Bulles fines Stable dans le temps (reste de la mousse après 2 heures) | Volume de mousse et taille des bulles faibles mais « mousse » stable dans le temps |
| CMC (g/L) | 6.0 | 0.24 | 0.58 |
| Tension superficielle à la CMC (mN/m) | 27.4 | 24.6 | 23.9 |
| Aire à l' interface (Å²) | 99 | 106 | / |

La comparaison des résultats obtenus en terme de tension interfaciale entre l'eau et l'huile de tournesol montre que l'augmentation de la longueur de la chaîne a une grande influence sur la tension interfaciale (C4 = 22.98 mN/m > C8 = 12.96 mN/m > C12 = 7.39 mN/m).

En ce qui concerne le pouvoir moussant, les résultats obtenus montrent qu'une longueur de chaîne optimale a été identifiée, c'est-à-dire deux chaînes en C8, permettant d"obtenir un volume de mousse relativement important (la mousse représente environ 1/3 du tube à essai de 5 mL) et des bulles fines ainsi qu'une mousse stable dans le temps (toujours présente après 2 heures même si elle diminue avec le temps). Pour les autres longueurs de chaîne, le pouvoir moussant n'est pas significatif.

La longueur de la chaîne a également une influence sur la CMC. En effet, l'augmentation de la longueur de la chaîne (de C4 à C8 ou C12 par exemple) permet de diminuer la CMC de 6.0 g/L à 0.24 g/L et 0.58 g/L respectivement. De plus dans tous les cas, la tension superficielle minimale est relativement basse, allant de 23.9 à 27.4 mN/m sans influence significative de la longueur de la chaîne. Dans la mesure où les composés obtenus selon l'invention sont des tensioactifs anioniques, il est possible de comparer ces valeurs avec un tensioactif anionique très connu, le sodium laureth sulfate (SLS) :

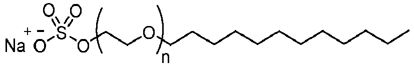

La CMC mesurée pour le SLS est de 0.14 g/L et la tension superficielle à la CMC est de 31.8 mN/m. D'après le tableau de résultats présentés précédemment, il apparaît que les produits obtenus selon l'invention présentent une tension superficielle plus basse que le SLS. Par contre, les CMC sont plus élévées. Pour faciliter la comparaison des produits obtenus selon l'invention avec le SLS, les concentrations permettant d'obtenir une tension superficielle de 31.8 mN/m ont été calculées : elles sont respectivement de 0.11 g/L et 0.19 g/L pour les composés en C8 et C12.

Il ressort de ce qui précède que les sels d'acide 5-dialkylacétal-2-furoïque obtenus selon l'invention présentent des propriétés intéressantes qui permettent leur utilisation dans des applications variées. Ces composés peuvent tous être utilisés dans des applications de détergence car la valeur de la tension superficielle minimale est relativement basse (allant de 23.9 à 27.4 mN/m) comparée au sodium laureth sulfate, un tensioactif anionique fort, largement utilisé dans l'industrie pour ses propriétés détergentes et moussantes. En particulier, la CMC est très intéressante dans le cas des molécules FurCO₂Na C₈C₈ et FurCO₂Na C₁₂C₁₂ (0.24g/L et 0.58 g/L respectivement) et le FurCO₂Na C₈C₈ présente en outre des propriétés moussantes intéressantes (volume important, bulles fines et stabilité de la mousse). De plus, les propriétés physico-chimiques du produit obtenu selon l'Exemple 14, contenant le FurCO₂Na C₈C₈, présente des valeurs de CMC (0.31 g/L) et de tension superficielle minimale (31.7 mN/m) relativement similaires à celles obtenus pour le produit pur.

Les autres molécules pourront donc être utilisées dans des produits détergents qui ne nécessitent pas un volume de mousse important.

## Revendications

1. Procédé de synthèse de 5-dialkylacétal-2-furoates d'alkyle de formule (I) : 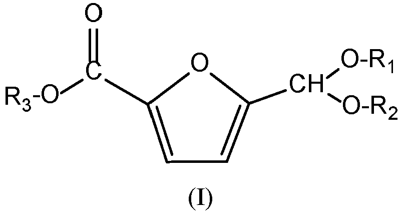
dans laquelle : R₁, R₂ et R₃ désignent chacun indépendamment un groupe alkyle ou alcényle en C₁-C₂₂,
**caractérisé en ce qu'**il comprend une étape de transformation d'un ou plusieurs acides uroniques par réaction avec au moins un alcool de formule R-OH où R désigne un groupe alkyle ou alcényle en C₁-C₂₂, en présence d'au moins un catalyseur acide, le milieu réactionnel étant porté à une température de 150 à 180°C.

2. Procédé selon la revendication 1, **caractérisé en ce que** les acides uroniques sont constitués d'un hydrolysat d'oligo- ou polysaccharides comprenant un ou plusieurs motifs identiques ou différents d'acide uronique éventuellement salifiés, et **en ce que** le procédé comprend en outre une étape d'hydrolyse acide réalisée simultanément à l'étape de transformation.

3. Procédé selon la revendication 1, **caractérisé en ce que** les acides uroniques sont constitués d'un hydrolysat d'oligo- ou polysaccharides comprenant un ou plusieurs motifs identiques ou différents d'acide uronique éventuellement salifiés, et **en ce que** le procédé comprend en outre une étape d'hydrolyse acide réalisée avant l'étape de transformation.

4. Procédé selon la revendication 2 ou 3, **caractérisé en ce que** l'étape de transformation est réalisée en présence du catalyseur acide introduit dans l'étape d'hydrolyse.

5. Procédé selon la revendication 3, **caractérisé en ce que** l'étape d'hydrolyse est suivie d'une étape de neutralisation puis d'élimination de l'eau, lesdites étapes étant réalisées successivement avant l'étape de transformation .

6. Procédé selon l'une quelconque des revendications 2 à 5, **caractérisé en ce que** les oligo- ou polysaccharides renferment au moins 40% molaire, de préférence au moins 70% molaire et, plus préférentiellement au moins 90% molaire de motifs acide uronique éventuellement associés à des motifs rhamnose, galactose et/ou glucose, et de préférence les oligo- et polysaccharides sont choisis parmi les alginates, les oligoalginates, les polymannuronates, les oligomannuronates, les polyguluronates, les oligoguluronates, les pectines, les oligopectines, les ulvanes et les oligoulvanes.

7. Procédé selon l'une quelconque des revendications 2 à 6, **caractérisé en ce que** l'alcool mis en oeuvre dans l'étape de transformation est de formule R-OH où R désigne un groupe alkyle ou alcényle en C₁-C₆ et **en ce qu'**il comprend en outre éventuellement une étape de transestérification et transacétalisation du composé de formule (I) avec un alcool R'-OH où R' représente un groupe alkyle ou alcényle en C₇-C₂₂.

8. Procédé selon l'une quelconque des revendications 1 à 7, **caractérisé en ce que** l'acide uronique est choisi parmi l'acide guluronique, l'acide glucuronique, l'acide galacturonique, l'acide mannuronique, l'acide iduronique, la glucurono-6,3-lactone et leurs mélanges.

9. Procédé selon la revendication 1, **caractérisé en ce qu'**il est mis en œuvre sur un ou plusieurs acides uroniques monomères et **en ce que** l'alcool mis en œuvre dans l'étape de transformation est de formule R-OH où R désigne un groupe alkyle ou alcényle en C₁-C₂₂.

10. Procédé selon l'une quelconque des revendications 1 à 9, **caractérisé en ce qu'**il comprend en outre une étape consistant à séparer le furoate d'alkyle co-produit avec le composé de formule (I).

11. Procédé selon l'une quelconque des revendications 1 à 10, **caractérisé en ce qu'**il comprend en outre une étape de saponification du produit de formule (I) ou du produit de transestérification et transacétalisation obtenu selon la revendication 7, pour obtenir des sels d'acide 5-dialkylacétal-2-furoïque de formule (II) : 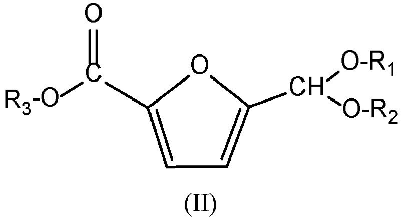 dans laquelle : R₁ et R₂ désignent chacun indépendamment un groupe alkyle ou alcényle en C₁-C₂₂ ; et R₃ désigne un atome de métal alcalin ou de métal alcalino-terreux ou un groupe ammonium quaternaire de formule (III) :
-N⁺-R₄R₅R₆R₇ (III)
où R₄, R₅, R₆ et R₇ désignent chacun indépendamment un atome d'hydrogène, un groupe alkyle en C₁-C₆ ou un groupe hydroxyalkyle en C₁-C₆.

12. Sels d'acide 5-dialkylacétal-2-furoïque de formule (II) : 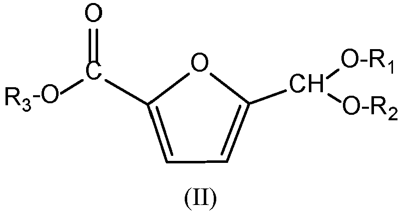 dans laquelle : R₁ et R₂ désignent chacun indépendamment un groupe alkyle ou alcényle en C₁-C₂₂ ; et R₃ désigne un atome de métal alcalin ou de métal alcalino-terreux ou un groupe ammonium quaternaire de formule (III) :
-N⁺-R₄R₅R₆R₇ (III)
où R₄, R₅, R₆ et R₇ désignent chacun indépendamment un atome d'hydrogène, un groupe alkyle en C₁-C₆ ou un groupe hydroxyalkyle en C₁-C₆.

13. Utilisation des sels d'acide 5-dialkylacétal-2-furoïque selon la revendication 12 comme agents tensioactifs.

14. Composition détergente renfermant les sels d'acide 5-dialkylacétal-2-furoïque selon la revendication 12.

## Patentansprüche

1. Verfahren zur Synthese von 5-Dialkylacetal-2-furoesäurealkylestern der Formel (I): 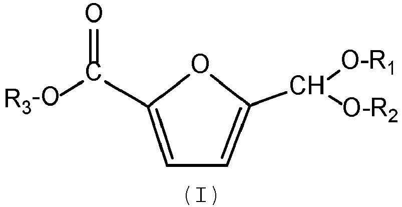
in der: R₁, R₂ und R₃ jeweils unabhängig für eine C₁-C₂₂-Alkyl- oder -Alkenylgruppe stehen,
**dadurch gekennzeichnet, dass** es einen Schritt der Umwandlung einer oder mehrerer Uronsäuren durch Umsetzung mit einem Alkohol der Formel R-OH, wobei R für eine C₁-C₂₂-Alkyl- oder -Alkenylgruppe steht, in Gegenwart mindestens eines sauren Katalysators umfasst, wobei das Reaktionsmedium auf eine Temperatur von 150 bis 180 °C gebracht wird.

2. Verfahren nach Anspruch 1, **dadurch gekennzeichnet, dass** die Uronsäuren aus einem Hydrolysat von Oligo- oder Polysacchariden, die eine oder mehrere gleiche oder verschiedene, gegebenenfalls versalzten Uronsäure-Einheiten umfassen, bestehen und dass das Verfahren außerdem einen Schritt der sauren Hydrolyse umfasst, der gleichzeitig mit dem Umwandlungsschritt durchgeführt wird.

3. Verfahren nach Anspruch 1, **dadurch gekennzeichnet, dass** die Uronsäuren aus einem Hydrolysat von Oligo- oder Polysacchariden, die eine oder mehrere gleiche oder verschiedene, gegebenenfalls versalzten Uronsäure-Einheiten umfassen, bestehen und dass das Verfahren außerdem einen Schritt der sauren Hydrolyse umfasst, der vor dem Umwandlungsschritt durchgeführt wird.

4. Verfahren nach Anspruch 2 oder 3, **dadurch gekennzeichnet, dass** der Umwandlungsschritt in Gegenwart des im Hydrolyseschritt eingetragenen sauren Katalysators durchgeführt wird.

5. Verfahren nach Anspruch 3, **dadurch gekennzeichnet, dass** auf den Hydrolyseschritt ein Schritt der Neutralisation mit anschließender Eliminierung von Wasser folgt, wobei die Schritte vor dem Umwandlungsschritt nacheinander durchgeführt werden.

6. Verfahren nach einem der Ansprüche 2 bis 5, **dadurch gekennzeichnet, dass** die Oligo- oder Polysaccharide mindestens 40 Mol-%, vorzugsweise mindestens 70 mol-% und weiter bevorzugt mindestens 90 mol-% Uronsäure-Einheiten, die gegebenenfalls mit Rhamnose-, Galactose- und/oder Glucose-Einheiten assoziiert sind, enthalten und die Oligo- und Polysaccharide vorzugsweise aus Alginaten, Oligoalginaten, Polymannuronaten, Oligomannuronaten, Polyguluronaten, Oligoguluronaten, Pektinen, Oligopektinen, Ulvanen und Oligoulvanen ausgewählt sind.

7. Verfahren nach einem der Ansprüche 2 bis 6, **dadurch gekennzeichnet, dass** der im Umwandlungsschritt eingesetzte Alkohol die Formel R-OH aufweist, wobei R für eine C₁-C₆-Alkyl- oder -Alkenylgruppe steht, und dass es außerdem gegebenenfalls einen Schritt der Umesterung und Umacetalisierung der Verbindung der Formel (I) mit einem Alkohol R'-OH, wobei R' für eine C₇-C₂₂-Alkyl- oder -Alkenylgruppe steht, umfasst.

8. Verfahren nach einem der Ansprüche 1 bis 7, **dadurch gekennzeichnet, dass** die Uronsäure aus Guluronsäure, Glucuronsäure, Galacturonsäure, Mannuronsäure, Iduronsäure, Glucurono-6,3-lacton und Mischungen davon ausgewählt wird.

9. Verfahren nach Anspruch 1, **dadurch gekennzeichnet, dass** es an einer oder mehreren monomeren Uronsäuren durchgeführt wird und dass der im Umwandlungsschritt eingesetzte Alkohol die Formel R-OH, wobei R für eine C₁-C₂₂-Alkyl- oder -Alkenylgruppe steht, aufweist.

10. Verfahren nach einem der Ansprüche 1 bis 9, **dadurch gekennzeichnet, dass** es außerdem einen Schritt umfasst, der aus der Abtrennung des zusammen mit der Verbindung der Formel (I) anfallenden Furoesäurealkylesters besteht.

11. Verfahren nach einem der Ansprüche 1 bis 10, **dadurch gekennzeichnet, dass** es außerdem einen Schritt der Verseifung des Produkts der Formel (I) oder des gemäß Anspruch 7 erhaltenen Umesterungs- und Umacetalisierungsprodukts zum Erhalt von 5-Dialkyl-acetal-2-furoesäuresalzen der Formel (II) umfasst: 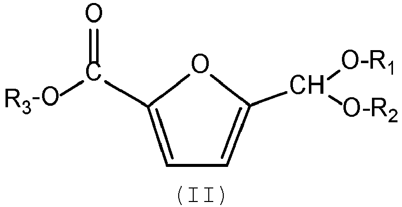 in der: R₁ und R₂ jeweils unabhängig für eine C₁-C₂₂-Alkyl- oder -Alkenylgruppe stehen und R₃ für ein Alkalimetall- oder Erdalkalimetallatom oder eine quartäre Ammoniumgruppe der Formel (III):
-N⁺-R₄R₅R₆R₇ (III)
stehen, wobei R₄, R₅, R₆ und R₇ jeweils unabhängig für ein Wasserstoffatom, eine C₁-C₆-Alkylgruppe oder eine C₁-C₆-Hydroxyalkylgruppe stehen.

12. 5-Dialkylacetal-2-furoesäuresalze der Formel: 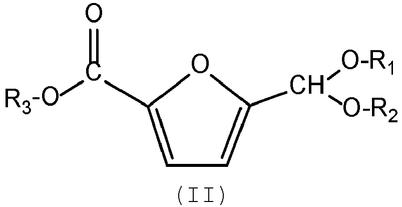 in der: R₁ und R₂ jeweils unabhängig für eine C₁-C₂₂-Alkyl- oder -Alkenylgruppe stehen und R₃ für ein Alkalimetall- oder Erdalkalimetallatom oder eine quartäre Ammoniumgruppe der Formel (III):
-N⁺-R₄R₅R₆R₇ (III)
stehen, wobei R₄, R₅, R₆ und R₇ jeweils unabhängig für ein Wasserstoffatom, eine C₁-C₆-Alkylgruppe oder eine C₁-C₆-Hydroxyalkylgruppe stehen.

13. Verwendung von 5-Dialkylacetal-2-furoesäuresalzen gemäß Anspruch 12 als Tenside.

14. Reinigungsmittel, das die 5-Dialkylacetal-2-furoesäuresalze gemäß Anspruch 12 enthält.

## Claims

1. Process for preparing alkyl 5-dialkylacetal-2-furoates of formula (I) : 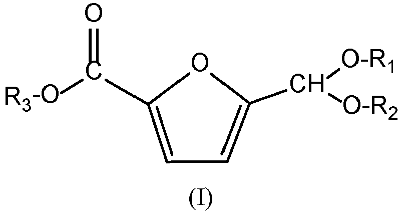 in which : R₁, R₂ and R₃ each independently designate a C₁-C₂₂ alkyl or alkenyl group, **characterized in that** it comprises a step of converting one or more uronic acids by reaction with at least one alcohol of formula R-OH wherein R designates a C₁-C₂₂ alkyl or alkenyl group, in the presence of at least one acidic catalyst, the reaction medium being brought to a temperature of 150 to 180°C.

2. Process according to claim 1, **characterized in that** the uronic acids consist of an hydrolysate of oligo- or polysaccharides comprising one or more identical or different moities of uronic acid which are optionally in salt form, and **in that** the process further comprises a step of acidic hydrolysis carried out simultaneously with the converting step.

3. Process according to claim 1, **characterized in that** the uronic acids consist of an hydrolysate of oligo- or polysaccharides comprising one or more identical or different moities of uronic acid which are optionally in salt form, and **in that** the process further comprises a step of acidic hydrolysis carried out before the converting step.

4. Process according to claim 2 or 3, **characterized in that** the converting step is carried out in the presence of the acidic catalyst which is introduced during the hydrolysis step.

5. Process according to claim 3, **characterized in that** the hydrolysis step is followed by a neutralizing step and a water removal step, said steps being carried out successively before the converting step.

6. Process according to any one of claims 2 to 5, **characterized in that** the oligo- or polysaccharides comprise at least 40 mol.%, preferably at least 70 mol.% and more preferably at least 90 mol.% of uronic acid moieties, optionally combined with rhamnose, galactose and/or glucose moieties, and preferably the oligo- and polysaccharides are selected from alginates, oligoalginates, polymannuronates, oligomannuronates, polyguluronates, oligoguluronates, pectines, oligopectines, ulvanes and oligoulvanes.

7. Process according to any one of claims 2 to 6, **characterized in that** the alcohol used in the converting step is of formula R-OH where R designates a C₁-C₆ alkyl or alkenyl group and **in that** it optionally further comprises a transesterification and transacetalisation step of the compound of formula (I) with a R'-OH alcohol where R' represents a C₇-C₂₂ alkyl or alkenyl group.

8. Process according to any one of claims 1 to 7, **characterized in that** the uronic acid is selected from guluronic acid, glucuronic acid, galacturonic acid, mannuronic acid, iduronic acid, glucurono-6,3-lactone and their mixtures.

9. Process according to claim 1, **characterized in that** it is performed on one or more uronic acid monomers and **in that** the alcohol used in the converting step is of formula R-OH where R designates a C₁-C₂₂ alkyl or alkenyl group.

10. Process according to any one of claims 1 to 9, **characterized in that** it further comprises a step consisting of separating the alkyl furoate co-produced with the compound of formula (I).

11. Process according to any one of claims 1 to 10, **characterized in that** it further comprises a step of saponifying the compound of formula (I) or the transesterification and transacetalisation product obtained according to claim 7, so as to obtain salts of 5-dialkylacetal-2-furoic acid of formula (II) : 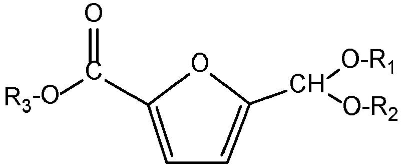 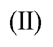 in which : R₁ and R₂ each independently designate a C₁-C₂₂ alkyl or alkenyl group; and R₃ designates an alkali metal or alkaline earth metal atom or a quaternary ammonium group of formula (III) :
-N⁺-R₄R₅R₆R₇ (III)
where R₄, R₅, R₆ and R₇ each independently designate a hydrogen atom, a C₁-C₆ alkyl group or a C₁-C₆hydroxyalkyl group.

12. Salts of 5-dialkylacetal-2-furoic acid of formula (II) : 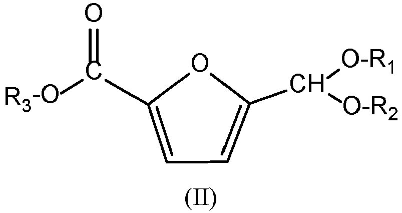 in which: R₁ and R₂ each independently designate a C₁-C₂₂ alkyl or alkenyl group; and R₃ designates an alkali metal or alkaline earth metal atom or a quaternary ammonium group of formula (III) :
-N⁺-R₄R₅R₆R₇ (III)
where R₄, R₅, R₆ and R₇ each independently designate a hydrogen atom, C₁-C₆ alkyl group or a C₁-C₆hydroxyalkyl group.

13. Use of the salts of 5-dialkylacetal-2-furoic acid according to claim 12 as surfactants.

14. Detergent composition comprising salts of 5-dialkylacetal-2-furoic acid according to claim 12.
